# EUROPÄISCHE PATENTSCHRIFT

(11) **EP 0 821 593 B1**
(45) Veröffentlichungstag und Bekanntmachung des Hinweises auf die Patenterteilung: **24.07.2002**
(21) Anmeldenummer: 96909047.1
(22) Anmeldetag: 12.04.1996
(51) Int. Cl.: A61K 47/48, A61P 17/00, A61P 31/00, A61P 35/00, A61P 37/00

(54) **KONJUGAT AUS EINEM WIRKSTOFF, EINEM POLYETHER UND GGF. EINEM NICHT ALS KÖRPERFREMD ANGESEHENEN, NATIVEN PROTEIN**
CONJUGATE OF AN ACTIVE AGENT, A POLYETHER AND POSSIBLY A NATIVE PROTEIN REGARDED AS ACCEPTABLE BY THE BODY
CONJUGUE COMPRENANT UN PRINCIPE ACTIF, UN POLYETHER ET EVENTUELLEMENT UNE PROTEINE A L'ETAT NATIF NON CONSIDEREE COMME EXOGENE

(30) Priorität: 13.04.1995 DE 19514087
(43) Veröffentlichungstag der Anmeldung: 04.02.1998
(73) Patentinhaber: Deutsches Krebsforschungszentrum Stiftung des öffentlichen Rechts, 69120 Heidelberg (DE)
(72) Erfinder: SINN, Hannsjörg, 69118 Wiesloch (DE); SCHRENK, Hans-Hermann, 67278 Zeiskamm (DE); MAIER-BORST, Wolfgang, 69221 Dossenheim (DE); STEHLE, Gerd, 68305 Mannheim (DE)
(74) Vertreter: Schüssler, Andrea, Dr.
(86) Internationale Anmeldenummer: DE9600653
(87) Internationale Veröffentlichungsnummer: WO9632134

(56) Entgegenhaltungen:
- EP-A- 0 473 084
- EP-A- 0 511 903
- EP-A- 0 576 192
- EP-A- 0 593 868
- WO-A-90/15059
- WO-A-95/29915
- DE-A- 4 017 439
- DE-A- 4 435 087
- US-A- 4 902 502
- BIOCONJUGATE CHEMISTRY, Bd. 4, Nr. 6, 1.November 1993, Seite 568/569 XP000417290 MIRON T ET AL: "A SIMPLIFIED METHOD FOR THE PREPARATION OF SUECINIMIDYL CARBONATE POLYETHYLENE GLYCOL FOR COUPLING TO PROTEINS"
- JOURNAL OF CONTROLLED RELEASE, Bd. 34, Nr. 3, 1.Juni 1995, Seiten 223-232, XP000505551 BONINA F P ET AL: "IN VIRTO AND IN VIVO EVALUATION OF POLYOXYLENE INDOMETHACIN ESTERS AS DERMAL PRODUGS"

## Beschreibung

Die Erfindung betrifft ein Konjugat aus einem Wirkstoff, einem Polyether und Albumin, Verfahren zur Herstellung eines solches Konjugats sowie dessen Verwendung.

Es ist seit langem ein Bedürfnis, Arzneimittel gezielt am Wirkort anzureichern. Insbesondere bei der Behandlung von Tumorerkrankungen, Infektionserkrankungen, Hauterkrankungen und/oder Erkrankungen des Immunsystems ist es wünschenswert, Arzneimittel in den von diesen Krankheiten befallenen Zellen und Geweben anzureichern. Desweiteren sollen die Arzneimittel eliminiert werden, wenn sie den Wirkort nicht erreichen. Damit soll die Belastung des Körpers durch die Arzneimittel minimiert werden.

WO-A-90/15059 beschreibt die Herstellung von Diels-Alder Porphyrinderivaten. Diese können ggf. an Rezeptor-spezifische Liganden oder spezifische Immunglobuline konjugiert sein.

Der vorliegenden Erfindung liegt somit die Aufgabe zugrunde, ein Mittel bereitzustellen, mit dem vorstehendes erreicht werden kann.

Erfindungsgemäß wird dies durch ein Konjugat aus einem Wirkstoff, einem Polyether und Albumin erreicht, das sich dadurch auszeichnet, daß die Komponenten über eine Säureamid- und/oder Säureesterbindung verbunden sind.

Nachstehend werden die Komponenten des erfindungsgemäßen Konjugats als Edukte dargestellt. Im erfindungsgemäßen Konjugat liegen sie in derivatisierter Form vor.

Der Ausdruck "Wirkstoff" umfaßt nachfolgende Verbindungen, die zur Behandlung und/oder Diagnose von Erkrankungen, wie Tumorerkrankungen, Infektionserkrankungen, Hauterkrankungen und/oder Erkrankungen des Immunsystems, verwendet werden können und die zur Ausbildung von Säureamid- und/oder Säureesterbindungen fähig sind.

Solche Verbindungen sind Chemotherapeutika, z.B. Antibiotika, Virusstatika, Antiprotozoenmittel und Zytostatika. Beispiele der Antibiotika sind Sulfonamide, Tetracycline, z.B. 7-Chlortetracyclin, Fusidinsäure, Gyrasehemmstoffe, z.B. Chinolone, Amphotericin, Isoniazid, Pyrazin-2-carbonsäure und Pyrazinamid. Beispiele der Virustatika sind Amantadin und Rimantadin. Beispiele von Antiprotozoenmittel sind Mefloquin und Primaquin. Beispiele für Zytostatika sind Anthrazykline, z.B. Doxorubicin, Topoisomerase-Hemmstoffe, Mitomycin A und C, Bleomycinsäure, Chlorambucil, Melphalan und Folsäureantagonisten, z.B. Methotrexat.

Weitere solche Verbindungen sind photoaktive Verbindungen, insbesondere eine photoaktive Verbindung, die in wäßrigen Medien photoaktiv ist. Beispiele solcher sind Porphyrine, wie o-, m-, und/oder p-Tetrahydroxyphenylporphin (THPP), o-, m- und/oder p-Tetracarboxyphenylporphin (TCPP) und o-, m-und/oder p-Tetrasulfophenylporphin, Chlorine, wie o-, m- und/oder p-Tetrahydroxyphenylchlorin, o-, m- und/oder p-Tetracarboxyphenylchlorin und o-, m-, und/oder p-Tetrasulfophenylchlorin, Bakteriochlorine, wie o-, m- und/oder p-Tetrahydroxyphenylbakteriochlorin, o-, m- und/oder p-Tetracarboxyphenylbakteriochlorin und o-, m-, und/oder p-Tetrasulfophenylbakteriochlorin, und Phtalocyanine, wie Phtalocyanintetrasulfonsäure. Vorstehende photoaktive Verbindungen können Metallionen, wie Al³⁺ oder Zn²⁺, aufweisen, die einen positiven Einfluß auf die Photoaktivität haben können.

Vorstehende Verbindungen können mit einer nachweisbaren Markierung, z.B. radioaktivem lod, einem Metallion, wie In³⁺, und/oder Gd³⁺, versehen sein. Dadurch können erfindungsgemäße Konjugate auch zur Diagnose von Erkrankungen verwendet werden.

Von vorstehenden Verbindungen bzw. Analoga oder Derivaten davon können in einem erfindungsgemäßen Konjugat eine oder mehrere vorliegen. Liegen mehrere vor, können diese gleich oder verschieden voneinander sein.

Ein erfindungsgemäßes Konjugat weist einen Polyether oder ein Derivat davon auf, die zur Ausbildung von Säureamid und/oder Säureesterbindungen fähig sind. Beispiele eines solchen Polyethers sind Polyethylenglykole (PEG), insbesondere solche, bei denen die terminalen Hydroxylgruppen mit einem C₁-C₁₂ Alkylrest, vorzugsweise Methyl, verestert oder verethert sind. Vertreter dieser sind Methoxypolyethylenglykol, Diaminomethoxypolyethylenglykol, Methoxyypolyethylenglykol-p-nitrophenylcarbonat, Methoxypolyethylenglykolsuccinimidylsuccinat, Methoxypolyethylenglykoltresylat, Methoxypolyoxyethylenamin (AminoPEG), Methoxypolyoxyethylencarbonsäureund Methoxypolyoxyethylenimidazolcarbonyl. Vorzugsweise weist der Polyether ein Molekulargewicht von 100 bis 20 000 Dalton, besonders bevorzugt etwa 5000 Dalton auf. Ferner kann der Polyether mit einer vorstehenden, nachweisbaren Markierung versehen sein.

In einem erfindungsgemäßen Konjugat liegen einer oder mehrere vorstehender Polyether vor. Bei mehreren können diese gleich oder verschieden voneinander sein.

In einem erfindungsgemäßen Konjugat liegt Albumin, insbesondere humanes Serumalbumin (HSA), vor.

Die Komponenten eines erfindungsgemäßen Konjugats werden so gewählt, daß das Molekulargewicht des Konjugats vorzugsweise mehr als ca. 10 000 Dalton und besonders bevorzugt mehr als ca. 15 000 Dalton beträgt.

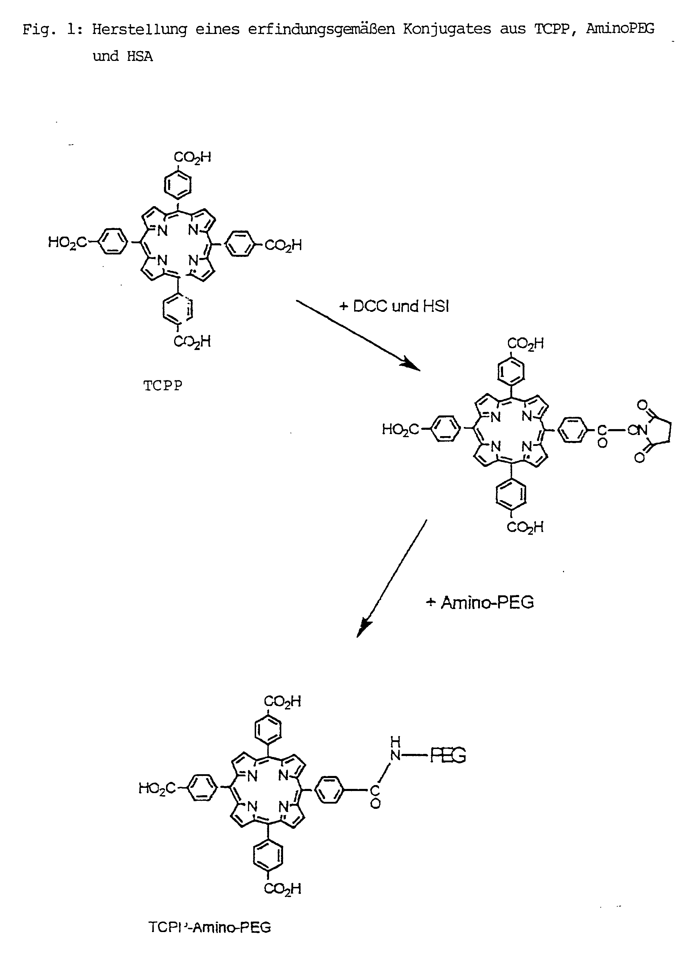

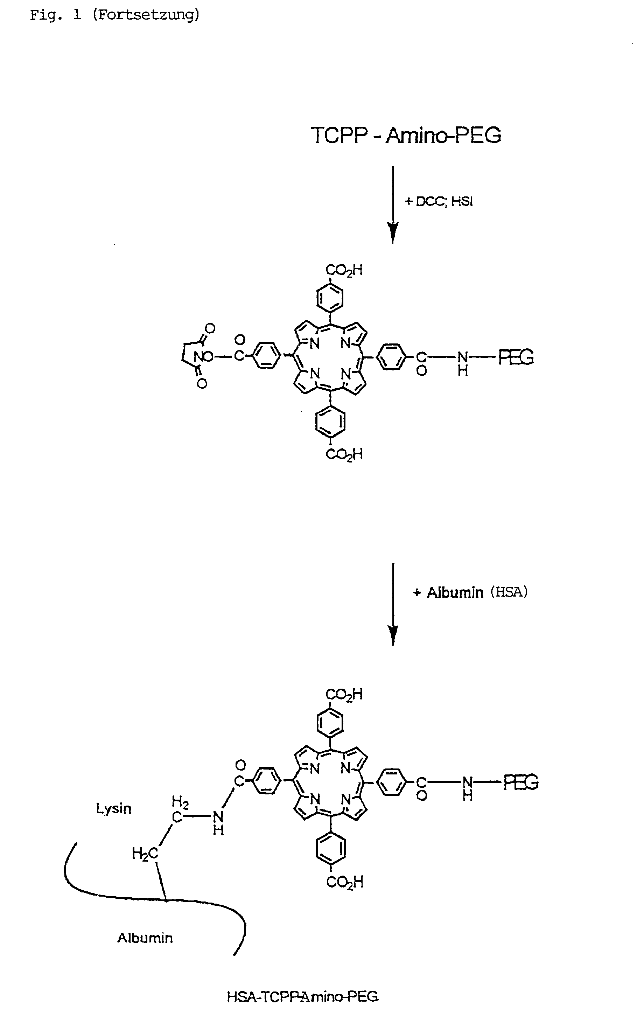

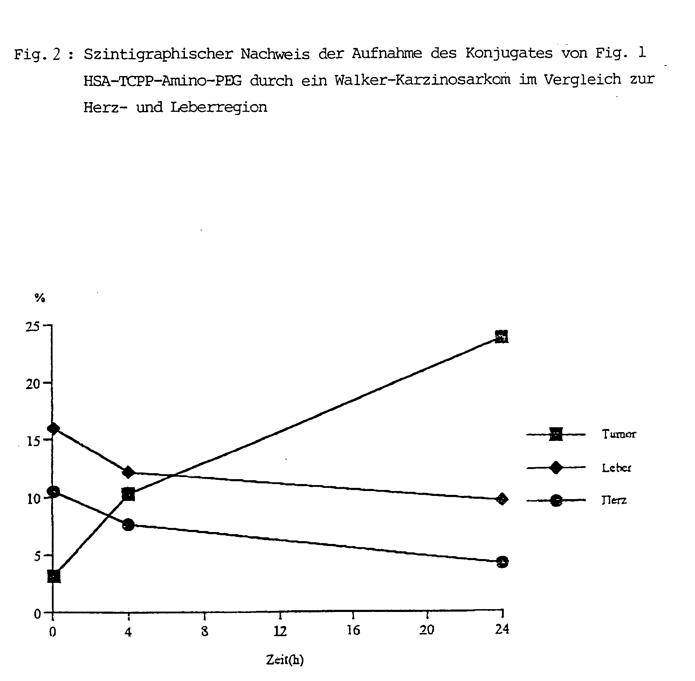

In einem erfindungsgemäßen Konjugat können die einzelnen Komponenten beliebig miteinander verbunden sein. Vorzugsweise ist der Wirkstoff sowohl an den Polyether als auch an Albumin gebunden (vgl. Figur 1).

Ein bevorzugtes erfindungsgemäßes Konjugat ist in Figur 1 dargestellt.

Erfindungsgemäß wird auch ein Verfahren zur Herstellung eines vorstehenden Konjugats bereitgestellt. In einem solchen Verfahren werden übliche Umsetzungen der Chemie durchgeführt, wie Aktivierung einer Säuregruppe und Verknüpfung der aktivierten Säuregruppe mit einer Amino- oder Hydroxylgruppe. Hierzu wird auf die Herstellung der Konjugate im Beispiel 1 sowie in der Figur 1 verwiesen.

Erfindungsgemäße Konjugate zeichnen sich dadurch aus, daß sie sich gezielt in bestimmten Zellen des Körpers anreichern, insbesondere Tumorzellen und Zellen von entzündlichem Gewebe und krankhaft veränderter Haut. Desweiteren zeichnen sich erfindungsgemäße Konjugate darin aus, daß sie schnell abgebaut werden, wenn sie den Wirkort nicht erreichen. Dadurch können Nebenwirkungen für den Körper minimiert werden.

Erfindungsgemäße Konjugate eignen sich somit bestens zur Behandlung von Erkrankungen, z.B. Tumorerkrankungen, Infektionserkrankungen, Hauterkrankungen, wie Psoriasis, und/oder Erkrankungen des Immunsystems.

Desweiteren können erfindungsgemäße Konjugate nach Markierung zur Diagnose von Erkrankungen, insbesondere vorstehender Erkrankungen, verwendet werden.

Kurze Beschreibung der Zeichnungen.
- Fig. 1: zeigt die Herstellung eines erfindungsgemäßen Konjugats aus TCPP, Amino PEG und HSA,
- Fig. 2: zeigt den szintigraphischen Nachweis der Aufnahme des Konjugats HSA-TCPP-AminoPEG (vgl. Fig. 1) durch ein Walker-Karzinosarkom im Vergleich zur Herz- und Leberregion.

Die folgenden Beispiele erläutern die Erfindung.

### Beispiel 1: Herstellung eines erfindungsgemäßen Konjugats aus TCPP, HSA und AminoPEG

Die Herstellung dieses Konjugats ist in Fig. 1 veranschaulicht.
a) TCPP-Aktivierung mit Dicyclohexylcarbodiimid (DCC) und Hydroxysuccinimid (HSI) und nachfolgende Umsetzung mit AminoPEG TCPP (FG:791) wird mit einer Konzentration von 10 mg/ml in Dimethylsulfoxid (DMSO) gelöst. Zu der klaren dunkelroten Lösung gibt man die vierfache molare Menge an Dicyclohexylcarbodiimid (DCC, FG:206,33) und die zehnfache molare Menge an Hydroxysuccinimid (HSI, FG: 115,1) hinzu. Nach etwa 2-3 h Reaktionszeit ist die Umsetzung zum TCPP-Succinimidylester (TCPP-SE) soweit fortgeschritten, daß nun die äquimolare Menge an AminoPEG zur Bildung von TCPP-AminoPEG hinzugegeben werden kann. Nach weiteren 12-14 h Reaktionszeit wird die Bildung von TCPP-AminoPEG-HSI₃ mit Hilfe einer DPC-Einheit geprüft.

| Laufbedingungen | | |
|---|---|---|
| HPLC-Pumpe, Rheodyne-Injektionsventil, UV-Monitor | | |
| Vorsäule | 30 x 8 mm, Eurogel SEC 1000, | |
| Säule | zwei 300 x 10 mm, Eurogel SEC 1000 | |
| | | |
| Lösungsmittel | DMF mit 1 mM LiBr (0,1 Mol% LiBr)/L DMF | |
| Fluß | 0,6 mL/min | |
| | | |

| Retentionszeiten | | |
|---|---|---|
| Polystyrolstandards | | |
| mittleres Molekulargewicht | 5kD | 27,00 min |
| | 10kD | 24,67 min |

b) Koppelung von TCPP-AminoPEG an humanes Serumalbumin (HSA): Zu einer Lösung von 4 g HSA_{(20%, Immuno)} (gelöst in 20 ml Originallösung + 20 ml 0,17 M NaHCO₃ und 15 ml EtOH) fügt man sehr langsam und unter ständigem Rühren die DMSO-Lösung des AminoPEG-TCPP-SE₃ hinzu, wobei sich die zunächst klare Lösung durch nicht umgesetztes DCC, das in wässeriger Lösung unlöslich ist, eintrübt. Nach Beendigung der Zugabe von AminoPEG-TCPP-SEₓ wird das Reaktionsgemisch zur möglichst vollständigen Umsetzung weitere 30 min bei Raumtemperatur gerührt und anschließend eine Stunde im Kühlschrank aufbewahrt. Anschließend wird die Trübung über eine Sterilfiltereinheit (Millipore, Stericup-GV, 0,22 µm Low Binding Duropore Membrane) und die niedermolekularen wasserlöslichen Komponenten (DMSO, HSI und nicht gebundenes TCPP) durch Ultrafiltration über eine Membrane mit 30 kD Ausschlußgrenze (Amicon YM 30) abgetrennt. Es wurde HSA-TCPP-AminoPEG erhalten (Fig. 1). Die Koppelungsausbeute von TCPP-AminoPEG an HSA beträgt im Durchschnitt 85-90%.
Die analytische Reinheitskontrolle erfolgt mittels HPLC.

| HPLC-Bedingungen: | |
|---|---|
| Vorsäule: | Zorbax Diol (50 x 4 mm) |
| Säule 1: | Zorbax GF 450, |
| Säule 2: | Zorbax GF 250 |
| Laufmittel: | 0,2 M Na-citrat, pH 7,5 |
| Fluß: | 1 ml/min |
| UV-Detektor 1: | 280 nm (für das Protein) |
| UV-Detektor 2: | 420 nm (für TCPP) |
| Aufgabevol.: | 100 µl (= maximal 10 mg Protein) |
| | |

| Retentionszeiten: | |
|---|---|
| dimere Fraktion | 17,7 min |
| monomere Fraktion | 19,77 min |

### Beispiel 2: Szintigraphischer Nachweis der Aufnahme des Konjugats HSA-TCPP-AminoPEG durch ein Walker-Karzinosarkom im Vergleich zur Herz- und Leberregion

Einer Ratte, die ein Walker-Karzinosarkom enthielt, wurde das erfindungsgemäße Konjugat HSA-TCPP-AminoPEG (vgl. Fig. 1) verabreicht. In üblicher Weise wurde szintigraphisch die Aufnahme des Konjugats im Tumor nachgewiesen und mit der Abnahme in der Herz- und Leberregion verglichen.

Wie aus Fig. 2 zu sehen ist, reichert sich das erfindungsgemäße Konjugat im Tumor an.

## Patentansprüche

1. Konjugat aus
- einem Wirkstoff ausgewählt aus einem Chemotherapeutikum oder einer photoaktiven Verbindung, welche zur Ausbildung einer Säureamidund/oder Säureesterbindung befähigt sind,
- einem Polyether, und
- Albumin,
**dadurch gekennzeichnet, daß** die Komponenten über eine Säureamid- und/oder Säureesterverbindung verbunden sind.

2. Konjugat nach Anspruch 1, **dadurch gekennzeichnet, daß** mehrere Wirkstoffe vorliegen.

3. Konjugat nach Anspruch 1 oder 2, **dadurch gekennzeichnet, daß** der Polyether ein Polyethylenglykol oder ein Derivat davon ist.

4. Konjugat nach einem der Ansprüche 1 - 3, **dadurch gekennzeichnet, daß** mehrere Polyether vorliegen.

5. Verfahren zur Herstellung eines Konjugats nach einem der Ansprüche 1-4, **dadurch gekennzeichnet, daß** ein Wirkstoff ausgewählt aus einem Chemotherapeutikum oder einer photoaktiven Verbindung, ein Polyether und Albumin unter Ausbildung von Säureamid- und/oder Säureesterverbindungen miteinander verbunden werden.

6. Verwendung eines Konjugats nach einem der Ansprüche 1 - 4 zur Herstellung eines Arzneimittels zur Behandlung einer Erkrankung.

7. Verwendung nach Anspruch 6, wobei die Erkrankung eine Tumorerkrankung, Infektionserkrankung, Hauterkrankung und/oder Erkrankung des Immunsystems ist.

## Claims

1. Conjugate from
- an active substance selected from a chemotherapeutic compound or a photoactive compound which are capable of forming an amide acid linkage or an ester acid linkage,
- a polyether, and
- albumin,
**characterised in that** the components are linked together by an amide acid linkage and/or an ester acid linkage.

2. Conjugate according to claim 1, **characterised in that** several active substances are present.

3. Conjugate according to claim 1 or 2, **characterised in that** the polyether is a polyethyleneglycol or a derivate thereof.

4. Conjugate according to one of claims 1-3, **characterised in that** several polyethers are present.

5. Method for the manufacture of a conjugate according to one of the claims 1-4, **characterised in that** an active substance selected from a chemotherapeutic compound or a photoactive compound, a polyether and albumin are linked together by the formation of amide acid linkage or ester acid linkage.

6. Use of a conjugate according to one of the claims 1-4 for the manufacture of a pharmaceutical agent for the treatment of an illness.

7. Use according to claim 6 wherein the illness is a tumour, an infection, a skin disease and/or an illness of the immune system.

## Revendications

1. Produit conjugué constitué
- d'une substance active choisie parmi un produit chimiothérapeutique ou un composé photo-actif, lesquels sont aptes à la formation d'une liaison amide d'acide et/ou ester d'acide,
- d'un polyéther et
- d'albumine,
**caractérisé en ce que** les composants sont reliés via une liaison amide d'acide et/ou ester d'acide.

2. Produit conjugué selon la revendication 1, **caractérisé en ce que** plusieurs substances actives sont présentes.

3. Produit conjugué selon la revendication 1 ou 2, **caractérisé en ce que** le polyéther est un polyéthylèneglycol ou un dérivé de celui-ci.

4. Produit conjugué selon l'une quelconque des revendications 1 à 3, **caractérisé en ce que** plusieurs polyéthers sont présents.

5. Procédé pour la fabrication d'un produit conjugué selon l'une quelconque des revendications 1 à 4, **caractérisé en ce qu'**une substance active choisie parmi un produit chimiothérapeutique ou un composé photo-actif, un polyéther et de l'albumine sont reliés l'un avec l'autre en formant des liaisons amide d'acide et/ou ester d'acide.

6. Utilisation d'un produit conjugué selon l'une quelconque des revendications 1 à 4 pour la fabrication d'un médicament pour le traitement d'une maladie.

7. Utilisation selon la revendication 6, la maladie étant une maladie tumorale, une maladie infectieuse, une maladie de la peau et/ou une maladie du système immunologique.
